# EUROPEAN PATENT APPLICATION

(11) **EP 4 509 050 A1**
(43) Date of publication of application: **19.02.2025**
(21) Application number: 23788258.4
(22) Date of filing: 06.04.2023
(51) Int. Cl.: A61B 5/16, A61B 5/02, A61B 5/11, G16H 50/30

(54) **INTERNAL STATE ESTIMATION DEVICE, INTERNAL STATE ESTIMATION METHOD, AND STORAGE MEDIUM**

(30) Priority: 13.04.2022 WO PCT/JP2022/017676
(71) Applicant: NEC Corporation, 108-8001 Tokyo (JP)
(72) Inventor: UMEMATSU, Terumi, Tokyo 108-8001 (JP); TSUJIKAWA, Masanori, Tokyo 108-8001 (JP)
(74) Representative: Betten & Resch
(86) International application number: PCT/JP2023/014240
(87) International publication number: WO 2023/199839

(57) **Abstract**

A mental state estimation device 1X mainly includes an observation data acquisition means 15X, a first mental state acquisition means 16X, and a second mental state estimation means 17X. The observation data acquisition means 15X acquires observation data obtained by observing a target person. The first mental state acquisition means 16X acquires an estimation result of a first mental state of the target person. The second mental state estimation means 17X estimates a second mental state of the target person, which is a mental state correlated with the first mental state, based on the observation data and the estimation result of the first mental state.

## Description

### TECHNICAL FIELD

The present disclosure relates to a technical field of a mental state estimation device, a mental state estimation method, and a storage medium that perform processing relating to estimation of a mental state.

### BACKGROUND

There are known devices or systems for estimating the mental state of a subject. For example, Patent Literature 1 discloses a system capable of determining the degree of concentration of a person shown in an image based on the image data by learning the relation between the image data of the user and the degree of concentration of the user.

### CITATION LIST

### PATENT LITERATURE

Patent Literature 1: JP2022-014473A

### SUMMARY

### PROBLEM TO BE SOLVED

When learning and estimating the mental state such as a degree of concentration by using a subjective value determined by human as a correct answer, the subjective value to be correct answer data includes a bias. It is difficult to prepare in advance such subjective values used as correct answer data, and there is an issue that sufficient training cannot be conducted with a small amount of data and a desired estimation accuracy cannot be obtained.

In view of the above-described issue, it is therefore an example object of the present disclosure to provide a mental state estimation device, a mental state estimation method, and a storage medium capable of accurately estimating the mental state.

### MEANS FOR SOLVING THE PROBLEM

In one mode of the mental state estimation device, there is provided a mental state estimation including:
an observation data acquisition means configured to acquire observation data obtained by observing a target person;
a first mental state acquisition means configured to acquire an estimation result of a first mental state of the target person; and
a second mental state estimation means configured to estimate a second mental state of the target person, which is a mental state correlated with the first mental state, based on the observation data and the estimation result of the first mental state.

In another mode of the mental state estimation method, there is provided a mental state estimation method executed by a computer, the mental state estimation method including:
acquiring observation data obtained by observing a target person;
acquiring an estimation result of a first mental state of the target person; and
estimating a second mental state of the target person, which is a mental state correlated with the first mental state, based on the observation data and the estimation result of the first mental state.

In one mode of the storage medium, there is provided a storage medium storing a program executed by a computer, the program causing the computer to
acquire observation data obtained by observing a target person;
acquire an estimation result of a first mental state of the target person; and
estimate a second mental state of the target person, which is a mental state correlated with the first mental state, based on the observation data and the estimation result of the first mental state.

### EFFECT

An example advantage according to the present invention is to accurately estimate the mental state of a target person.

### BRIEF DESCRIPTION OF THE DRAWINGS

[FIG. 1] It illustrates a schematic configuration of a mental state estimation system according to the first example embodiment.
[FIG. 2] It illustrates a hardware configuration of an information processing device.
[FIG. 3] FIG. 3A illustrates a distribution diagram of the second mental state whose axis is the sleepiness that is the first mental state, in such a case where the sleepiness is set as the first mental state and the degree of concentration is set as the second mental state.
FIG. 3B illustrates a distribution diagram of the second mental state whose axis is the tension level that is the first mental state, in such a case where the tension level is set as the first mental state and the pleasure-displeasure scale is set as the second mental state.
[FIG. 4] It illustrates an example of a functional block diagram of the mental state estimation device.
[FIG. 5] It illustrates an example of a detailed functional block diagram of the first mental state estimation unit and the second mental state estimation unit.
[FIG. 6] It illustrates an example of the functional block diagram of the processor of the mental state estimation device regarding the learning of an estimation model.
[FIG. 7] It illustrates an example of a detailed functional block of the second learning unit.
[FIG. 8] It illustrates an example of a flowchart showing a processing procedure related to the estimation of the mental state by the mental state estimation device according to the first example embodiment.
[FIG. 9] It illustrates an example of a detailed function block of the second learning unit according to the first modification.
[FIG. 10] It illustrates an example of a detailed function block of the second mental state estimation unit according to the first modification.
[FIG. 11] It illustrates a schematic configuration of a mental state estimation system according to the second example embodiment.
[FIG. 12] It illustrates a block diagram of a mental state estimation device according to the third example embodiment.
[FIG. 13] It illustrates an example of a flowchart executed by the mental state estimation device according to the third example embodiment.

### EXAMPLE EMBODIMENTS

Hereinafter, example embodiments of a mental state estimation device, a mental state estimation method, and a storage medium will be described with reference to the drawings.

### <First Example Embodiment>

### (1) System Configuration

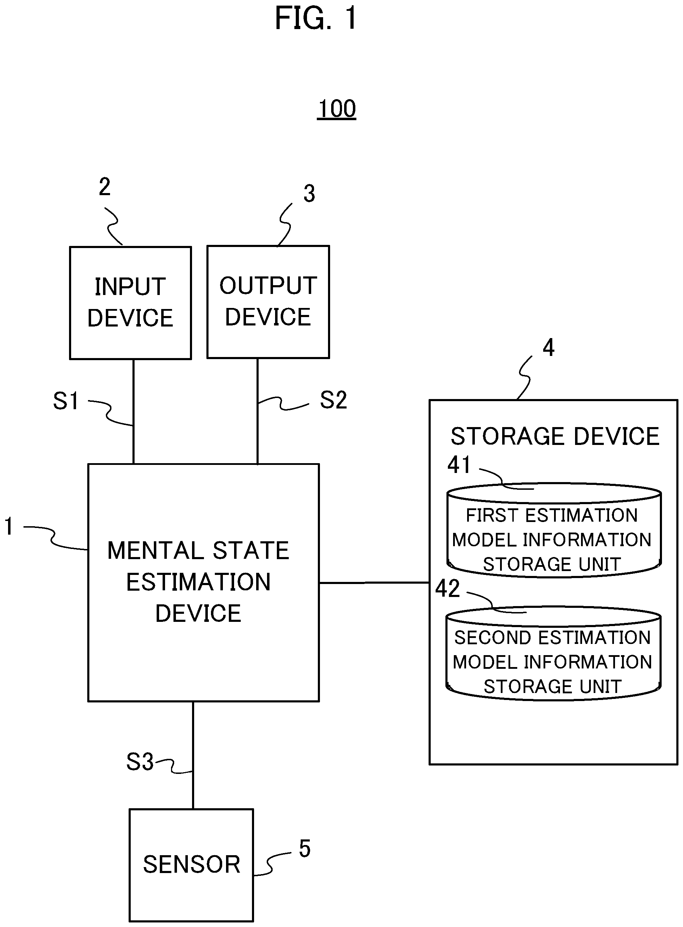

FIG. 1 shows a schematic configuration of a mental state estimation system 100 according to the first example embodiment. The mental state estimation system 100 highly accurately estimates the mental state of a target person such as the degree of concentration that cannot be accurately determined only by observation from others, and presents the estimation result. Here, the "target person" may be a target person of management of the mental state by an organization, or may be an individual user.

The mental state estimation system 100 mainly includes a mental state estimation device 1, an input device 2, an output device 3, a storage device 4, and a sensor 5.

The mental state estimation device 1 performs data communication with the input device 2, the output device 3, and the sensor 5 through a communication network or through wireless or wired direct communication. The mental state estimation device 1 estimates the mental state of the target person based on an input signal "S1" supplied from the input device 2, a sensor (detection) signal "S3" supplied from the sensor 5, and the information stored in the storage device 4. Based on the estimation result of the mental state of the target person (also referred to as the "first mental state") which is observed as an appearance of the physiological phenomenon of the target person, the mental state estimation device 1 estimates a second mental state with high accuracy. The "second mental state" is a mental state in which a state that is observed as an appearance of the physiological phenomenon of the target person and a state that is not observed as an appearance of the physiological phenomenon of the target person are mixed. The "second mental state" cannot be judged or it is difficult to be judged only by an observation by others (in other words, it cannot be judged except for an expert). Specific examples of the first mental state and the second mental state will be described later. The mental state estimation device 1 generates an output signal "S2" relating to the estimation result of the second mental state of the target person and supplies the generated output signal S2 to the output device 3. The mental state estimation device 1 may perform processing relating to the learning of the estimation model of the mental state to be described later.

The input device 2 is one or more user interfaces that receive manual input (external input) of information regarding each target person. The user who inputs the information using the input device 2 may be the target person itself, or may be a person who manages or supervises the activity of the target person. The input device 2 may be a variety of user input interfaces such as, for example, a touch panel, a button, a keyboard, a mouse, and a voice input device. The input device 2 supplies the generated input signal S1 to the mental state estimation device 1. The output device 3 displays or outputs predetermined information based on the output signal S2 supplied from the mental state estimation device 1. Examples of the output device 3 include a display, a projector, and a speaker.

The sensor 5 measures a biological signal regarding the target person and supplies the measured biological signal to the mental state estimation device 1 as a sensor signal S3. In this instance, the sensor signal S3 may be any biological signal (including vital information) regarding the target person such as a heart rate, EEG, pulse wave, sweating volume (skin electrical activity), amount of hormonal secretion, cerebral blood flow, blood pressure, body temperature, myoelectric potential, respiration rate, and acceleration. The sensor 5 may also be a device that analyzes blood collected from the target person and outputs a sensor signal S3 indicative of the analysis result. Examples of the sensor 5 include a wearable terminal worn by the target person, a camera for photographing the target person, a microphone for generating a voice signal of the target person's utterance, and a terminal such as a personal computer or a smartphone operated by the target person. For example, the above-described wearable terminal includes a GNSS (global navigation satellite system) receiver, an acceleration sensor, a sensor for detecting biological signals, and the like, and outputs the output signals from each sensor as a sensor signal S3. The sensor 5 may supply information corresponding to the manipulation amount signal from a personal computer or a smartphone to the mental state estimation device 1 as the sensor signal S3. The sensor 5 may also output a sensor signal S3 representing biomedical data (including sleep time) regarding the target person during the sleep.

The storage device 4 is a memory for storing various information necessary for processing performed by the mental state estimation device 1. The storage device 4 may be an external storage device, such as a hard disk, connected to or embedded in the mental state estimation device 1, or may be a storage medium, such as a flash memory. The storage device 4 may be a server device that performs data communication with the mental state estimation device 1. Further, the storage device 4 may be configured by a plurality of devices.

The storage device 4 functionally includes a first estimation model information storage unit 41 and a second estimation model information storage unit 42.

The first estimation model information storage unit 41 stores information regarding a first estimation model, which is a model for estimating the first mental state. The first estimation model is a model configured to output a first mental state estimate value representing the estimation result of the first mental state of a target person in response to the input of data (e.g., a feature vector) in a predetermined tensor format representing an observation result of the target person. The model is, for example, any machine learning model (including a statistical model, hereinafter the same) such as a neural network and a support vector machine, and the parameters necessary to configure the first estimation model are stored in the first estimation model information storage unit 41. For example, when the above-described first estimation model is a model based on a neural network such as a convolutional neural network, the first estimation model information storage unit 41 stores information regarding various parameters such as the layer structure, the neuron structure of each layer, the number of filters and the filter size in each layer, and the weight for each element of each filter. The first mental state estimate value is not necessarily a scalar value, and it may be a vector value including a plurality of numerical values.

The second estimation model information storage unit 42 stores information regarding a second estimation model, which is a model for estimating the second mental state. The second estimation model is a model configured to output the second mental state estimate value representing the estimation result of the second mental state of a target person in response to the input of data (for example, a feature vector) in a predetermined tensor format representing the target person's observation result is inputted thereto. The model is, for example, any machine learning model (including a statistical model, hereinafter the same) such as a neural network and a support vector machine, and the parameters necessary to configure the second estimation model are stored in the second estimation model information storage unit 42. It is noted that the data inputted to the second estimation model includes the first mental state estimate value outputted by the first estimation model. The second mental state estimate is not necessarily a scalar value, and it may be a vector value including a plurality of values.

The data to be inputted to the first estimation model and the second estimation model is not limited to data based on the observation result of a target person observed at a certain time, and it may be data based on the observation results in time series of the target person during a predetermined period.

In addition, when the mental state estimation device 1 performs learning of at least one of the first estimation model and/or the second estimation model, training data necessary for learning of the first estimation model or/and the second estimation model is further stored in the storage device 4. The training data will be described later.

The configuration of the mental state estimation system 100 shown in FIG. 1 is an example, and various changes may be made to the configuration. For example, the input device 2 and the output device 3 may be configured integrally. In this case, the input device 2 and the output device 3 may be configured as a tablet-type terminal that is incorporated into or separate from the mental state estimation device 1. Further, the input device 2 and the sensor 5 may be configured integrally. Further, the mental state estimation device 1 may be configured by a plurality of devices. In this case, the plurality of devices constituting the mental state estimation device 1 transmits and receives information necessary for executing the preassigned process among the plurality of devices. In this case, the mental state estimation device 1 functions as a system.

### (2) Hardware Configuration

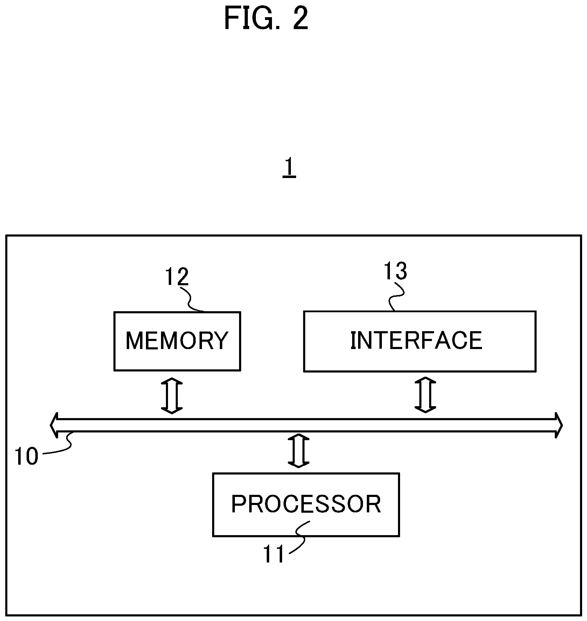

FIG. 2 shows a hardware configuration of the mental state estimation device 1. The mental state estimation device 1 includes a processor 11, a memory 12, and an interface 13 as hardware. The processor 11, the memory 12 and the interface 13 are connected to one another via a data bus 10.

The processor 11 executes a program stored in the memory 12 and thereby functions as a controller (arithmetic unit) for performing overall control of the mental state estimation device 1. Examples of the processor 11 include a CPU (Central Processing Unit), a GPU (Graphics Processing Unit), and a TPU (Tensor Processing Unit). The processor 11 may be configured by a plurality of processors. The processor 11 is an example of a computer.

The memory 12 is configured by a variety of volatile and non-volatile memories, such as a RAM (Random Access Memory), a ROM (Read Only Memory), and a flash memory. Further, the memory 12 stores a program for the mental state estimation device 1 to execute a process. A part of the information stored in the memory 12 may be stored by one or more external storage devices capable of communicating with the mental state estimation device 1, or may be stored in a storage medium detachable from the mental state estimation device 1.

The interface 13 is one or more interfaces for electrically connecting the mental state estimation device 1 to other devices. Examples of these interfaces include a wireless interface, such as a network adapter, for transmitting and receiving data to and from other devices wirelessly, and a hardware interface, such as a cable, for connecting to other devices.

The hardware configuration of the mental state estimation device 1 is not limited to the configuration shown in FIG. 2. For example, the mental state estimation device 1 may include at least one of the input device 2 and/or the output device 3. Further, the mental state estimation device 1 may be connected or incorporate a sound output device such as a speaker.

### (3) Specific Example of First Mental State and Second Mental State

The first mental state has a correlation with the second mental state. Then, the first mental state is an expressed mental state that is visually recognized by the other, the second mental state is a state in which the expressed mental state that is visually recognized by the other and the mental state subjectively recognized by only himself or herself are mixed.

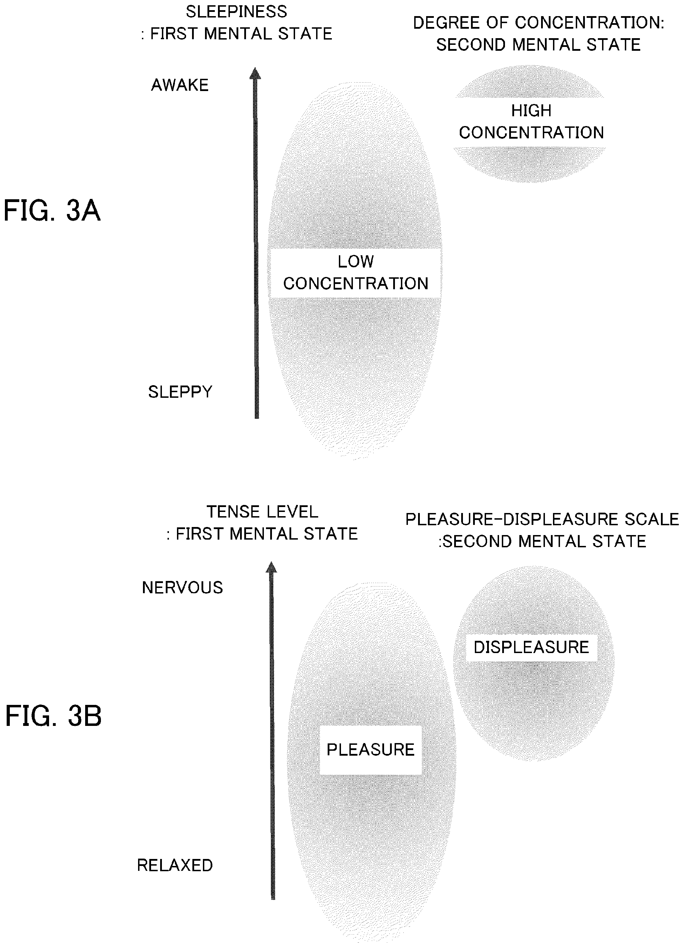

FIGS. 3A and 3B is a diagram conceptually showing a relation between the first mental state and the second mental state. FIG. 3A shows a distribution diagram of the second mental state with the axis "sleepiness" set as the first mental state when the first mental state is "sleepiness" and the second mental state is "degree of concentration". Further, FIG. 3B shows a distribution diagram of the second mental state with the axis "tension level" set as the first mental state when the first mental state is "tension level" and the second mental state is "pleasure-displeasure scale". As shown in FIG. 3A, the state of high concentration occurs in a state where there is little sleepiness, while the state of low concentration (i.e., a low concentration state) is widely distributed with respect to the axis of the first mental state "sleepiness". As a result, the state of high concentration and the state of low concentration are mixed in the state of less sleepiness. Further, as shown in FIG. 3B, while the state of displeasure occurs in a nervous state (i.e., state of high tension), the state of pleasure is widely distributed with respect to the axis of the first mental state "tense level". As a result, the state of pleasure and the state of displeasure are mixed in the nervous state. Thus, the first mental state has a correlation with the second mental state, and the second mental state includes all or a part of the first mental state.

Here, a description will be given of a specific example of the first mental state in the case of estimating the degree of concentration as the second mental state. For example, the first mental state may be "sleepiness". In this situation, the first estimation model is a model configured to estimate the sleepiness, and outputs a NEDO index (sleepiness label) as the first mental state estimate value. In another example, the first mental state may be "emotion". In this case, the first estimation model is a model configured to estimate the emotion regarding delight, anger, sorrow and pleasure, and outputs an index value indicating the emotion as the first mental state estimate value. In yet another example, the first mental state may be a combination of "arousal" and "pleasure-displeasure scale". In this case, the first estimation model is configured to output an index value based on the Russell's circumplex model, for example, as the first mental state estimate value.

Next, a description will be given of examples of the combination of the first mental state and the second mental state in the case where the second mental state is other than the degree of concentration. For example, if the first mental state is "tension level", then the second mental state is "pleasure-displeasure scale". In another example, when the first mental state is "sleepiness", the second mental state is any one of "degree of arousal", "tension level", "degree of health", "activity level", and "degree of anxiety". In yet another example, if the first mental state is "emotion", the second mental state is any one of "degree of health", "activity level", and "mood". In yet another example, if the first mental state is "pleasure-displeasure scale", the second mental state is either "mood" or "emotion".

The correct answer data to be used for training the first estimation model configured to estimate the first mental state indicates an objective evaluation value (objective evaluation value) annotated (i.e., labeled as the correct answer) by a person other than a subject observed in the generation of the input data to the first estimation model. On the other hand, the correct answer data used in the second estimation model for estimating the second mental state indicates the subjective evaluation value (subjective evaluation value) by a subject itself observed in the generation of the input data of the first estimation model. For example, the correct answer data to be used in the second estimation model indicates the answer result of the questionnaire for subjective value of the second mental state. For example, if the second mental state is the degree of concentration, the correct answer data indicates the concentration level indicated by the questionnaire result (e.g., on a scale of one to five). Further, as will be described later, the input data to the first estimation model may be a part of the input data to the second estimation model.

### (4) Functional Blocks

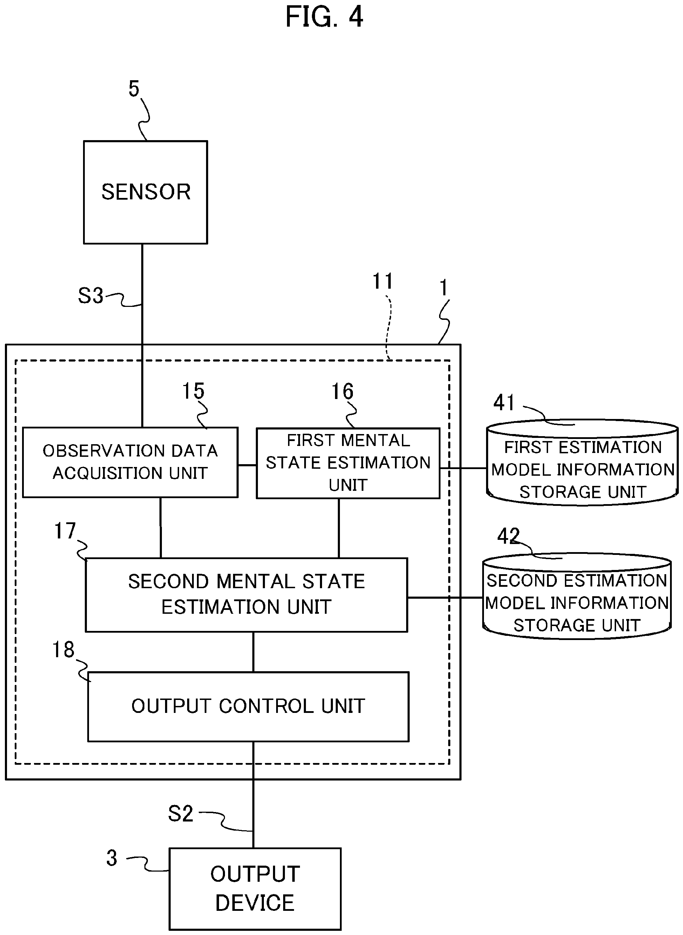

FIG. 4 is an example of a functional block diagram of the mental state estimation device 1. The processor 11 of the mental state estimation device 1 functionally includes an observation data acquisition unit 15, a first mental state estimation unit 16, a second mental state estimation unit 17, and an output control unit 18. In FIG. 4, blocks to exchange data with each other are connected by a solid line, but the combination of blocks to exchange data with each other is not limited to FIG. 4. The same applies to the drawings of other functional blocks described below.

The observation data acquisition unit 15 receives the sensor signal S3 obtained by observing the target person at a target time of estimation or during a target period of estimation of the mental state of the target person from the sensor 5 via the interface 13 and acquires the observation data based on the received sensor signal S3. The above-described target time or target period of the estimation may be the latest time point or period at intervals of a predetermined time, or may be a pre-specified time point or period. Further, the observation data may be the sensor signal S3 itself, or may be data obtained through a predetermined signal process on the sensor signal S3. Then, the observation data acquisition unit 15 extracts data (also referred to as "first observation data") to be used for the estimation of the first mental state from the observation data, and supplies the first observation data to the first mental state estimation unit 16. The observation data acquisition unit 15 extracts data (also referred to as "second observation data") to be used for estimation of the second mental state from the observation data and supplies the second observation data to the second mental state estimation unit 17. In addition to the sensor signal S3 supplied from the sensor 5, the observation data acquisition unit 15 may generate observation data using the input signal S1 supplied from the input device 2.

The first observation data herein is data to be used for estimation of the first mental state, and for example, the first observation data is physiological information representing an index value related to the observed physiological phenomenon of the target person such as heart rate and amount of perspiration. In another example, the first observation data is any data (non-contact sensor data) obtained from a non-contact sensor which does not contact with the target person. In this case, the first observation data may be facial data (e.g., video data of the face of the target person or an index value, such as a facial expression and a lid opening degree, calculated from the video data) that is information regarding the face of the target person, or may be voice data (including an index value calculated from the voice signal) representing a voice of the target person. In yet another example, the first observation data may be motion data relating to the movement of the target person. The motion data may be, for example, video data of the target person in a walking state or an index value calculated from the video data, or may be an observed acceleration of the target person. Further, the first observation data may be a combination of the above-described pieces of information. The first observation data may be any kind of observation data to be used for estimation of the target first mental state of estimation. If the first observation data is a predetermined index value or the like, the observation data acquisition unit 15 performs a process of generating the first observation data from the sensor signal S3 based on a program or the like stored in the memory 12 or the storage device 4.

The second observation data is data to be used for estimation of the second mental state. Examples of the second observation data include physiological information regarding the target person, non-contact sensor data such as face data and voice data, motion data, and a combination thereof. The second observation data may be any kind of observation data to be used for estimation of the second mental state to be estimated. When the second observation data is a predetermined index value, the observation data acquisition unit 15 generates the second observation data from the sensor signal S3 based on a program or the like stored in the memory 12 or the storage device 4.

It is noted that the first observation data and the second observation data may be partially-overlapped data, or may be separated data. For example, when the first mental state is "sleepiness" and the second mental state is "degree of concentration", the first observation data is lid opening degree and blinking information based on a facial video of the target person, and the second observation data is lid opening degree, blinking information, facial expression information, and line-of-sight information based on the facial video of the target person.

The first mental state estimation unit 16 estimates the first mental state of the target person based on the first observation data supplied from the observation data acquisition unit 15 and supplies a first mental state estimate value, which represents an estimation result of the first mental state, to the second mental state estimation unit 17. In this case, the first mental state estimation unit 16 inputs the first observation data or the feature values (which may be a feature vector and hereinafter the same) based on the first observation data to the first estimation model configured by referring to the first estimation model information storage unit 41, and acquires a first mental state estimate value outputted by the first estimation model in response to the input.

The second mental state estimation unit 17 estimates the second mental state of the target person based on the second observation data supplied from the observation data acquisition unit 15 and the first mental state estimate value supplied from the first mental state estimation unit 16. The second mental state estimation unit 17 supplies the second mental state estimate value, which is a value representing the estimation result of the second mental state, to the output control unit 18. In this case, the first mental state estimation unit 16 inputs the first mental state estimate value and either the second observation data or the feature values based on the second observation data to the second estimation model configured by referring to the second estimation model information storage unit 42, and acquires a second mental state estimate value outputted by the second estimation model in response to the input.

The output control unit 18 outputs information relating to the estimation result of the mental state of the target person. For example, the output control unit 18 displays the estimation result of the mental state (specifically, the second mental state) of the target person generated by the second mental state estimation unit 17 on the display unit of the output device 3, or, outputs it by the sound output unit of the output device 3. In this case, the output control unit 18 may output, together with the estimation result of the second mental state, the estimation result of the first mental state.

In some embodiments, the output control unit 18 compares the estimation result of the mental state with a reference value (criterion) for determining the presence or absence of abnormality of the mental state, and notifies the target person or the manager of a predetermined information based on the comparison result. For example, if the estimation result of the mental state indicates a value worse than the reference value, the output control unit 18 outputs a predetermined warning information, or may output information relating to the advice.

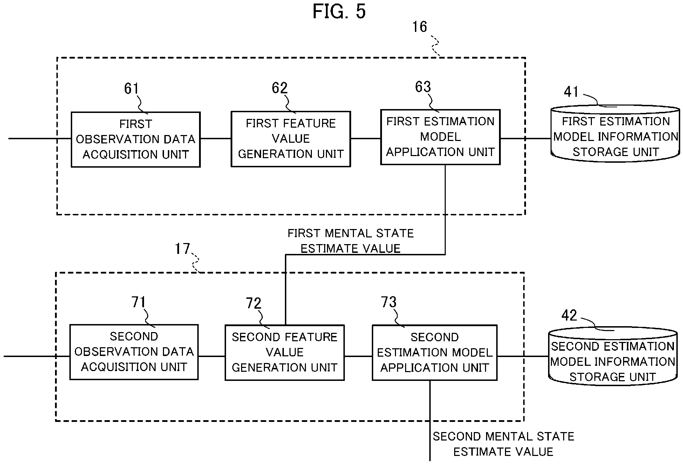

FIG. 5 is an example of a detailed functional block diagram of the first mental state estimation unit 16 and the second mental state estimation unit 17. In the example shown in FIG. 5, the first mental state estimation unit 16 functionally includes a first observation data acquisition unit 61, a first feature value generation unit 62, and a first estimation model application unit 63. The second mental state estimation unit 17 functionally includes a second observation data acquisition unit 71, a second feature value generation unit 72, and a second estimation model application unit 73.

The first observation data acquisition unit 61 acquires the first observation data from the observation data acquisition unit 15 and supplies the acquired first observation data to the first feature value generation unit 62. The first feature value generation unit 62 generates first feature values which are feature values to be inputted to the first estimation model. In this case, the first feature value generation unit 62 generates the feature values of the first observation data acquired by the first observation data acquisition unit 61 as the first feature values. The first feature values are data in a predetermined tensor format which is an input format of the first estimation model to be used by the first estimation model application unit 63. The feature extraction process which the first feature generation unit 62 performs may be based on any feature extraction technique. For example, the feature extraction process executed by the first feature value generation unit 62 may be a process of calculating statistics such as the average, variance, and the like of the index values included in the first observation data, or may be a process of acquiring the feature values outputted by a feature extraction model, which is a learned deep learning model, in response to the input of the first observation data into the feature extraction model.

The first estimation model application unit 63 inputs the first feature values supplied from the first feature value generation unit 62 to the first estimation model configured by referring to the first estimation model information storage unit 41, and acquires a first mental state estimate value outputted by the first estimation model in response to the input. Then, the first estimation model application unit 63 supplies the acquired first mental state estimate value to the second feature value generation unit 72.

The second observation data acquisition unit 71 acquires the second observation data from the observation data acquisition unit 15 and supplies the acquired second observation data to the second feature value generation unit 72. The second feature value generation unit 72 generates second feature values which are feature values to be inputted to the second estimation model. In this case, first, the second feature value generation unit 72 generates feature values of the second observation data acquired by the second observation data acquisition unit 71. Then, the second feature value generation unit 72 generates the second feature values that is feature values obtained by adding the first mental state estimate value supplied from the first estimation model application unit 63 to the generated feature values. The second feature values are data in a predetermined tensor format that conforms to the input format of the second estimation model used by the second estimation model application unit 73. The feature extraction process of the second observation data by the second feature generation unit 72 may be a process based on any feature extraction technique.

The second estimation model application unit 73 inputs the second feature values supplied from the second feature value generation unit 72 to the second estimation model configured by referring to the second estimation model information storage unit 42, and acquires the second mental state estimate value outputted by the second estimation model in response to the input. Then, the second estimation model application unit 73 supplies the acquired second mental state estimate value to the output control unit 18.

According to the configuration shown in FIGS. 4 and 5, the mental state estimation device 1 can estimate the second mental state of the target person with high accuracy based on the observation data of the target person and the estimation result of the first mental state. The mental state estimation device 1 can suitably facilitate the self-management of the target person for productivity improvement by outputting such a simply and accurately estimation result of the second mental state such as the degree of concentration.

Here, for example, each component of the observation data acquisition unit 15, the first mental state estimation unit 16, the second mental state estimation unit 17 and the output control unit 18 as described in FIG. 4 can be realized by the processor 11 executing a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as a FPGA (Field-Programmable Gate Array) and microcontrollers. In this case, the integrated circuit may be used to realize a program for configuring each of the above-described components. Further, at least a part of the components may be configured by an ASSP (Application Specific Standard Produce), an ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology. The above explanation is also true for other embodiments to be described below.

### (5) Model Learning

Next, a learning method of the first estimation model and the second estimation model will be described. Hereafter, as an example, a case in which the mental state estimation device 1 performs the learning of the first estimation model and the second estimation model will be described. However, one or more devices other than the mental state estimation device 1 may perform the learning of the first estimation model and the second estimation model. In addition, the learning of the first estimation model and the learning of the second estimation model may be performed by separate devices, respectively. For example, the mental state estimation device 1 may perform the learning of the second estimation model (that is, processing of the second learning unit 22 to be described later) by referring to parameters of the first estimation model which has already been trained by another device. In the following, one or more persons who are subjected to observation in the generation of the training data are also referred to as "subject". The target person may be plural, and may include the target person or not include the target person.

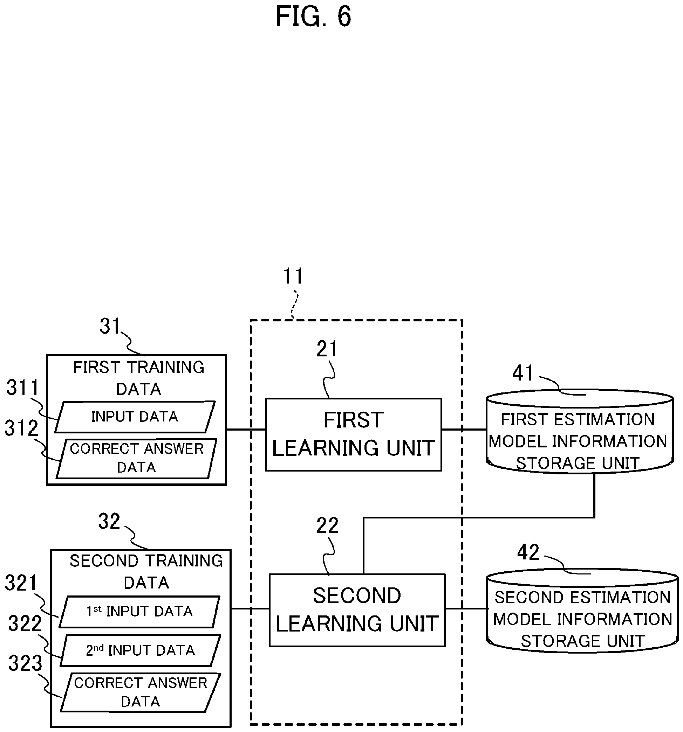

FIG. 6 is an example of the functional block diagram of the processor 11 of the mental state estimation device 1 relating to the learning of the first estimation model and the second estimation model. Regarding learning of the first estimation model and the second estimation model, the processor 11 functionally includes a first learning unit 21 and a second learning unit 22. Further, the storage device 4 stores first training data 31 and second training data 32.

The first training data 31 is training data to be used for learning of the first estimation model and includes input data 311 and correct answer data 312. The input data 311 corresponds to a predetermined number of records of the first observation data prepared in advance, and the correct answer data 312 indicates the correct answer of the first mental state estimate value to be outputted by the first estimation model for each record of the input data 311. Here, since the first mental state is the expressed mental state that others can virtually recognize, the correct answer data 312 is generated by annotation work executed by person(s) other than the subject of the input data 311. In other words, the correct answer data 312 is generated based on the objective evaluation, and indicates objective evaluation values of the first mental state of the subject at the observing time point of each record of the input data 311.

The first learning unit 21 trains the first estimation model based on the input data 311 and the correct answer data 312, and stores the parameters of the first estimation model obtained by the training in the first estimation model information storage unit 41. In this case, the first learning unit 21 acquires the first mental state estimate value outputted by the first estimation model when one selected record of the input data 311 or its feature values (i.e., feature values corresponding to the first feature values) is inputted to the first estimation model. Then, the first learning unit 21 updates the parameters of the first estimation model such that the error (loss) between the first mental state estimate value outputted by the first estimation model and the correct answer indicated by the correct answer data 312 corresponding to the used record of the input data 311 is minimized. Then, the first learning unit 21 executes this process to all records of the input data 311 or until a predetermined learning end condition is satisfied. The algorithm for determining parameters to minimize the loss may be any learning algorithm used in machine learning, such as the gradient descent method and the error back propagation method. The initial parameters of the first estimation model may be stored in the first estimation model information storage unit 41 before the learning of the first estimation model.

The second training data 32 is training data to be used for learning of the second estimation model and includes first input data 321, second input data 322, and correct answer data 323. The first input data 321 corresponds to a predetermined number of records of the first observation data prepared in advance. The second input data 322 corresponds to a predetermined number of records of the second observation data prepared in advance, which are obtained by observing the same subject at the same timing (i.e., the same time) as the records of the first input data 321, respectively. The correct answer data 323 indicates the correct answer of the second mental state estimate value to be outputted by the second estimation model for each record set of the first input data 321 and the second input data 322 obtained at the same observation timing regarding the same subject. Here, since the second mental state is the mental state of the subject that cannot be accurately determined by others only based on the appearance, the correct answer data 323 is generated by annotation work (e.g., questionnaire result, etc.,) by the subject of the first input data 321 and the second input data 322. In other words, the correct answer data 323 is data generated based on the subjective evaluation and indicates the subjective evaluation value of the second mental state of the subject at each observation point in each record set of the first input data 321 and the second input data 322.

The second learning unit 22 trains the second estimation model based on the parameters of the learned first estimation model stored in the first estimation model information storage unit 41 and the second training data 32, and stores the parameters of the second estimation model obtained by the training in the second estimation model information storage unit 42.

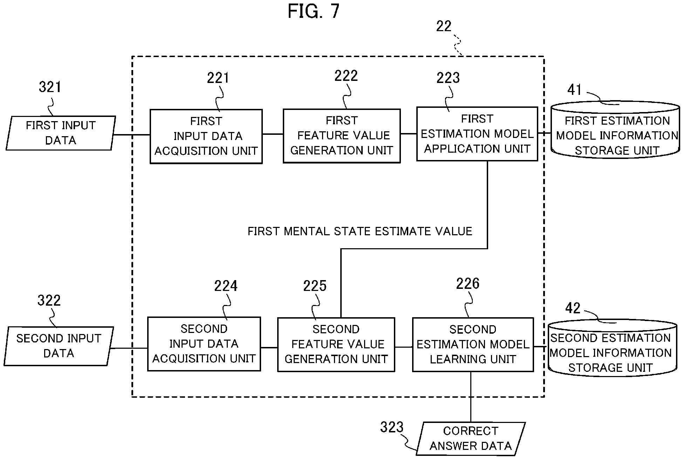

FIG. 7 is an example of a detailed functional block diagram of the second learning unit 22. The second learning unit 22 shown in FIG. 7 functionally includes a first input data acquisition unit 221, a first feature value generation unit 222, a first estimation model application unit 223, a second input data acquisition unit 224, a second feature value generation unit 225, and a second estimation model learning unit 226.

The first input data acquisition unit 221 extracts a record of the first input data 321 to which the first estimation model is to be applied from the second training data 32. The first feature value generation unit 222 generates first feature values, which conform to the input format of the first estimation model, from the record of the first input data 321 obtained by the first input data acquisition unit 221. The feature value extraction process executed by the first feature value generation unit 222 uses the same algorithm as the feature value extraction process executed by the first feature value generation unit 62. The first estimation model application unit 223 inputs the first features value generated by the first feature value generation unit 222 to the first estimation model configured based on the parameters of the learned first estimation model stored in the first estimation model information storage unit 41. Then, the first estimation model application unit 223 supplies the second feature value generation unit 225 with the first mental state estimate value outputted by the first estimation model in response to the input of the first feature value.

The second input data acquisition unit 224 acquires a record of the second input data 322 corresponding to (i.e., obtained at the same observation timing regarding the same subject as) the record of the first input data 321 obtained by the first input data acquisition unit 221. The second feature value generation unit 225 generates the second feature values which conform to the input format of the second estimation model based on the record of the second input data 322 acquired by the second input data acquisition unit 224 and the first mental state estimate value supplied from the first estimation model application unit 223. The feature extraction process executed by the second feature value generation unit 225 uses the same algorithm as the feature extraction process executed by the second feature value generation unit 72.

The second estimation model learning unit 226 inputs the second feature values generated by the second feature value generation unit 225 to the second estimation model configured based on the parameters stored in the second estimation model information storage unit 42 and acquires a second mental state estimate value outputted by the second estimation model in response to the input. Then, the second estimation model learning unit 226 updates the parameters of the second estimation model such that the error (loss) between the second mental state estimate value obtained by the second estimation model and the correct answer indicated by the correct answer data 323 corresponding to the first input data 321 and the second input data 322 used for generating of the second feature values is minimized. Then, the second learning unit 22 executes this process for all record sets of the first input data 321 and the second input data 322 or until a predetermined learning end condition is satisfied. The algorithm for determining parameters to minimize the loss may be any learning algorithm used in machine learning, such as the gradient descent method and the error back propagation method. The initial parameters of the second estimation model may be stored in the second estimation model information storage unit 42 before learning of the second estimation model.

As described above, in the above-described learning method, the mental state estimation device 1 uses the output from the first estimation model trained based on the first training data 31 that can be generated (i.e., can be easily obtained in a large amount) by objective evaluation by person(s) other than the subject as a part of input to the second estimation model. Thus, the mental state estimation device 1 can train the second estimation model configured to estimate the second mental state with high accuracy by using the second training data 32 (i.e., a relatively small amount of data) which needs subjective evaluation by the subject.

### (6) Processing Flow

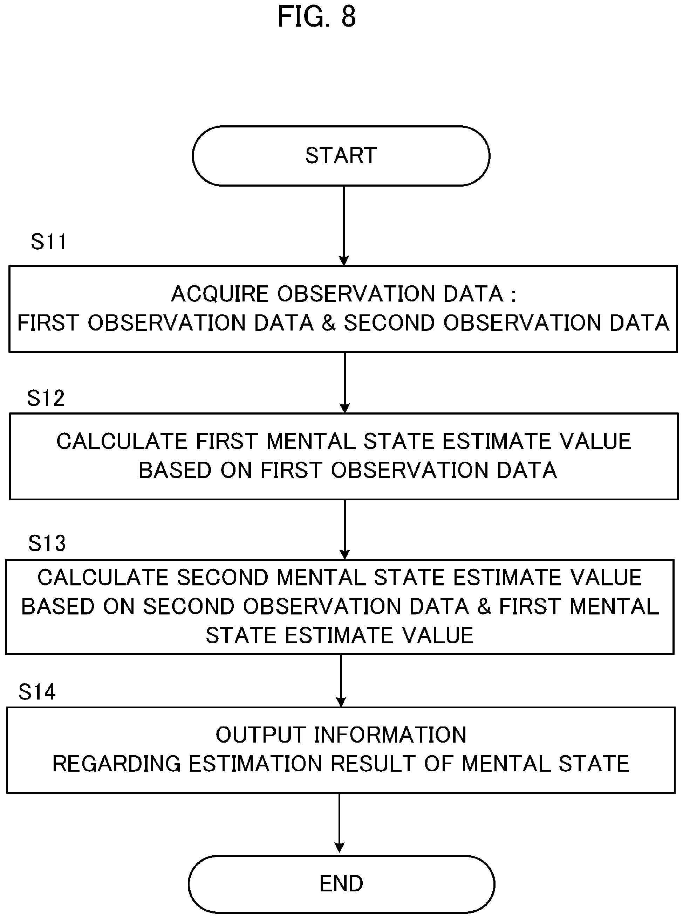

FIG. 8 is an example of a flowchart illustrating a processing procedure relating to estimation of the mental state executed by the mental state estimation device 1 after learning of the first estimation model and the second estimation model.

For example, if a predetermined estimation execution condition of the mental state is satisfied, the mental state estimation device 1 determines that it is the timing of estimating the mental state and executes the process of the flowchart in FIG. 8. For example, if the mental state estimation device 1 receives the input signal S1 for instructing the execution of the estimation process of the mental state from the input device 2, the mental state estimation device 1 determines that the above-described estimation execution condition is satisfied. The mental state estimation device 1 may refer to the estimation execution condition stored in advance in the storage device 4 to determine whether or not the estimation execution condition is satisfied. The mental state estimation device 1 may determine that the estimation execution condition is satisfied if it is a predetermined date and time set in advance (e.g., a predetermined time on every day). In yet another example, the mental state estimation device 1 may determine that the estimation execution condition has been met if it acquires the sensor signal S3 for generating the observation data required for estimation of the mental state.

First, the mental state estimation device 1 acquires the observation data obtained by observing a target person at the above-described estimation timing of the mental state (step S11). In this instance, the mental state estimation device 1 acquires the observation data based on the sensor signal S3 acquired from the sensor 5 through the interface 13. Furthermore, the mental state estimation device 1 extracts the observation data to be used for the first estimation model from the acquired observation data as the first observation data, and extracts the observation data to be used for the second estimation model from the acquired observation data as the second observation data. It is noted that the first observation data and the second observation data may overlap with each other.

Then, the mental state estimation device 1 calculates the first mental state estimate value based on the first observation data acquired at step S11 (step S12). In this case, the mental state estimation device 1 calculates the first mental state estimate value based on the first observation data and the first estimation model, which is configured by referring to the learned parameters stored in the first estimation model information storage unit 41.

Next, the mental state estimation device 1 calculates the second mental state estimate value based on the second observation data acquired at step S11 and the first mental state estimate value calculated at step S12 (step S13). In this case, the mental state estimation device 1 calculates the second mental state estimate value based on the first mental state estimate value and the second estimation model, which is configured by referring to the learned parameters stored in the second estimation model information storage unit 42.

Next, the mental state estimation device 1 outputs the estimation result of the mental state (step S14). The estimation result of the mental state is not limited to the second mental state estimate value and it may include the first mental state estimate value. In this instance, the mental state estimation device 1 supplies the output signal S2 to the output device 3 so that the output device 3 performs a display or audio output representing the estimation result of the mental state. In this case, in some embodiments, the output control unit 18 compares the estimation result of the mental state with a predetermined reference value, and then notify, based on the comparison result, the target person or the manager which manages the target person of information regarding the estimation result of the mental state. Thus, the mental state estimation device 1 can suitably present information regarding the estimation result of the mental state of the target person to the target person or its manager.

In some embodiments, if the second mental state is the degree of concentration, the mental state estimation device 1 may output information as to whether or not the task which the target person worked on is the task performed with concentration. Further, the mental state estimation device 1 may handle the task, which was performed with the concentration whose degree was less than a predetermined degree, differently (e.g., nullifying the test result when the task is a test) from the task performed with the concentration whose degree was equal to or larger than a predetermined degree.

### (7) Modifications

A description will be given of preferred modifications to the example embodiment described above. The modifications may be applied to the above example embodiment in any combination.

### (First Modification)

The mental state estimation device 1 may use the correct answer data 312 of the first training data 31 instead of using the first mental state estimate value outputted by the first estimation model as a part of the input to the second estimation model.

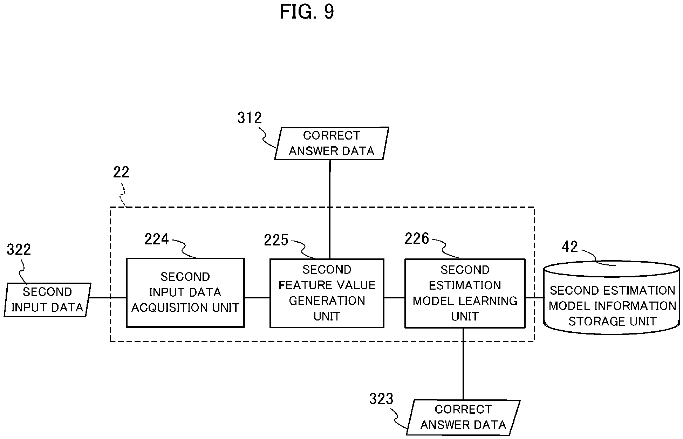

FIG. 9 is an example of a detailed function block diagram of the second learning unit 22 according to the first modification. The second learning unit 22 according to the first modification functionally includes a second input data acquisition unit 224, a second feature value generation unit 225, and a second estimation model learning unit 226. Here, the second input data 322 corresponds to the second observation data observed from the same subject at the same timing as the correct answer state indicated by the correct answer data 312.

The second input data acquisition unit 224 extracts one record of the second input data 322 to be inputted to the second estimation model from the second training data 32. The second feature value generation unit 225 generates the second feature values based on the record of the second input data 322 acquired by the second input data acquisition unit 224 and the correct answer data 312 indicating the correct answer value of the first mental state at the time of the observation of the second input data 322. The second estimation model learning unit 226 inputs the second feature values generated by the second feature value generation unit 225 to the second estimation model configured based on the parameters stored in the second estimation model information storage unit 42. Then, the second estimation model learning unit 226 updates the parameters of the second estimation model such that the error (loss) between the second mental state estimate value outputted by the second estimation model and the correct answer indicated by the correct answer data 323 corresponding to the second input data 322 used for generating the second feature values is minimized.

In this case, in some embodiments, even in the estimation of the mental state of the target person, the mental state estimation device 1 may estimate the second mental state using the correct answer data 312 indicating the correct answer of the first mental state of the target person at the time of the observation of the second observation data, instead of the first mental state estimate value outputted by the first estimation model.

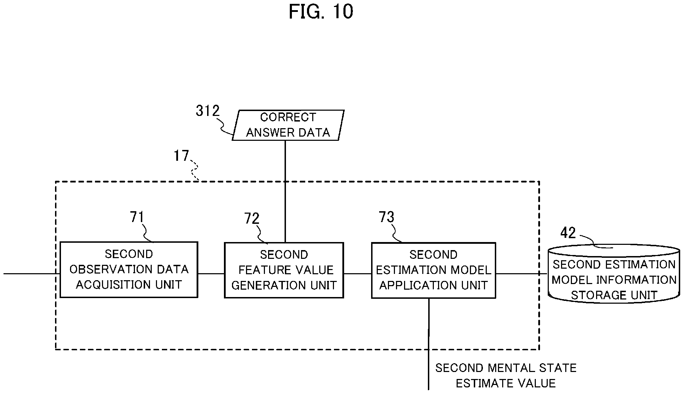

FIG. 10 is an example of a detailed function block diagram of the second mental state estimation unit 17 according to the first modification. The second mental state estimation unit 17 according to the first modification functionally includes a second observation data acquisition unit 71, a second feature value generation unit 72, and a second estimation model application unit 73.

The second observation data acquisition unit 71 acquires the second observation data from the observation data acquisition unit 15 and supplies the acquired second observation data to the second feature value generation unit 72. It is noted that the second observation data is data obtained by observing the target person in the past and it is data observed at the same time as the first mental state of the target person indicated by the correct answer data 312.

The second feature value generation unit 72 generates the second feature values that is obtained by adding the correct answer of the first mental state indicated by the correct answer data 312 to the feature values extracted from the second observation data acquired by the second observation data acquisition unit 71. The second estimation model application unit 73 inputs the second feature values supplied from the second feature value generation unit 72 to the second estimation model configured by referring to the second estimation model information storage unit 42, and acquires the second mental state estimate value outputted by the second estimation model in response to the input. Then, the second estimation model application unit 73 supplies the acquired second mental state estimate value to the output control unit 18. The correct answer of the first mental state indicated by the correct answer data 312 is an example of the estimation result of the first mental state of the target person.

Thus, the mental state estimation device 1 according to the first modification can suitably execute learning of the second estimation model and estimation of the second mental state using the correct answer data 312.

### (Second Modification)

The first mental state and the second mental state may be mental states represented by a common index. For example, the first mental state and the second mental state are both represented by an index relating to emotion (joy, anger, sorrow, and pleasure), wherein the first mental state is an expressed state of the emotion, and the second mental state is an inner (non-expressed) mental state of the emotion.

In this case, the first mental state estimation unit 16 sets the mental state to be estimated as an expressed first mental state visually recognized by others, and calculates the first mental state estimate value based on the first estimation model that is trained by using an objective evaluation value as correct answer data. On the other hand, the second mental state estimation unit 17 sets the mental state to be estimated as the second mental state of the target person, which cannot be accurately determined by others only based on the appearance, and calculates the second mental state estimate value based on the second estimation model trained by using a subjective evaluation value as correct solution data. In addition, in the learning, similarly to the above-described example embodiment, the first learning unit 21 trains the first estimation model using correct answer data indicating an objective evaluation value, and the second learning unit 22 trains the second estimation model using correct answer data indicating a subjective evaluation value. Thus, such a learned second estimation model configured to estimate the second mental state with high accuracy is obtained.

According to this modification, the mental state estimation device 1 can calculate the second mental state estimate value representing the mental state to be estimated with high accuracy.

### <Second Example Embodiment>

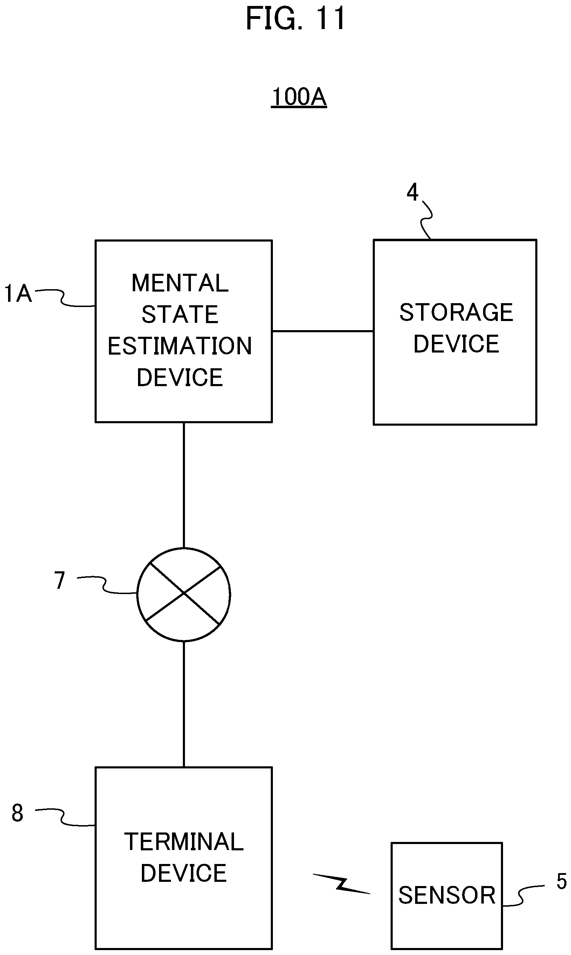

FIG. 11 shows a schematic configuration of a mental state estimation 100A according to a second example embodiment. The mental state estimation system 100A according to the second example embodiment is a system of a server-client model, and the mental state estimation device 1A functioning as a server device performs a process which the mental state estimation device 1 executed in the first example embodiment. Hereinafter, the same components as those in the first example embodiment are appropriately denoted by the same reference numerals, and a description thereof will be omitted.

The mental state estimation 100A mainly includes the mental state estimation device 1A that functions as a server, a storage device 4 which stores the same data as in the first example embodiment, and a terminal device 8 that functions as a client. The mental state estimation device 1A and the terminal device 8 performs data communication with each other via the network 7.

The terminal device 8 is a terminal equipped with an input function, a display function, and a communication function, and functions as the input device 2 and the output device 3 shown in FIG. 1. Examples of the terminal device 8 include a personal computer, a tablet-type terminal, and a PDA (Personal Digital Assistant). The terminal device 8 transmits a biological signal outputted by sensor (not shown) or an input signal based on the user input to the mental state estimation device 1A.

For example, the mental state estimator 1A has the same hardware configuration and functional configuration as the mental state estimation device 1. The mental state estimation device 1A receives information, which the mental state estimation device 1 shown in FIG. 1 obtains from the input device 2 and the sensor 5, from the terminal device 8 via the network 7. Then, the mental state estimation device 1 A estimates the mental state of the target person based on the received information. The mental state estimation device 1A transmits an output signal indicating the information regarding the above-described estimation result to the terminal device 8 through the network 7 in response to a request from the terminal device 8. That is, in this case, the terminal device 8 functions as the output device 3 in the first example embodiment. Thus, the mental state estimation device 1A suitably presents the user of the terminal device 8 with information relating to the estimation result of the mental state.

### <Third Example Embodiment>

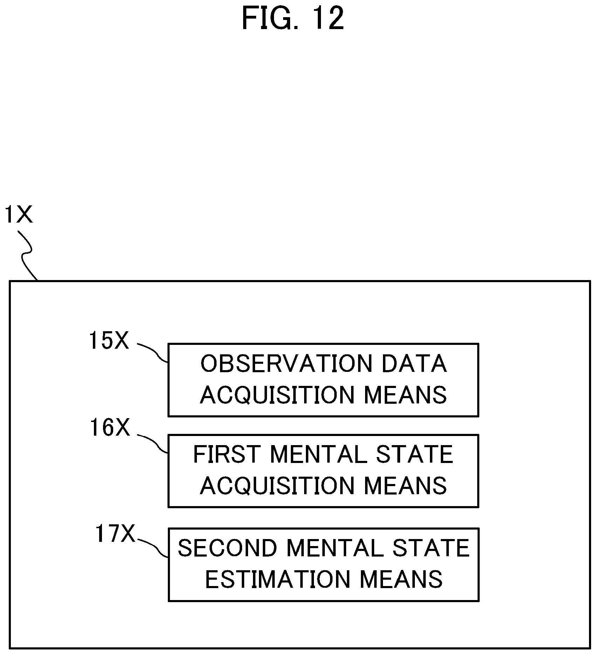

FIG. 12 is a block diagram of a mental state estimation device 1X according to the third example embodiment. The mental state estimation device 1X mainly includes an observation data acquisition means 15X, a first mental state acquisition means 16X, and a second mental state estimation means 17X. The mental state estimation device 1X may be configured by a plurality of devices.

The observation data acquisition means 15X is configured to acquire observation data obtained by observing a target person. Examples of the observation data acquisition means include the observation data acquisition unit 15 according to the first example embodiment (including modifications, hereinafter the same) or the second example embodiment.

The first mental state acquisition means 16X is configured to acquire an estimation result of a first mental state of the target person. Examples of the first mental state acquisition means 16X include the first mental state estimation unit 16 in the first example embodiment (excluding the first modification) or the second example embodiment. Examples of the first mental state acquisition means 16X also include the second mental state estimation unit 17 that acquires the correct answer data 312 as the estimation result of the first mental state in the first modification of the example embodiment.

The second mental state estimation means 17X is configured to estimate a second mental state of the target person, which is a mental state correlated with the first mental state, based on the observation data and the estimation result of the first mental state. Examples of the second mental state estimation means 17X include the second mental state estimation unit 17 in the first example embodiment or the second example embodiment.

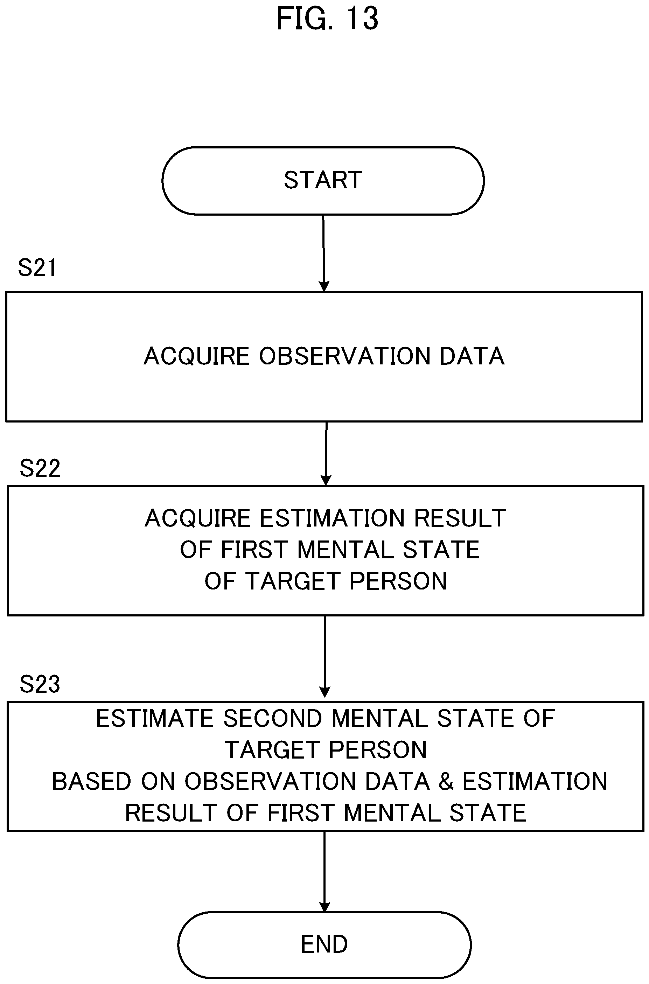

FIG. 13 is an exemplary flowchart that is executed by the mental state estimation device 1X in the third example embodiment. The observation data acquisition means 15X acquires observation data obtained by observing a target person (step S21). The first mental state acquisition means 16X acquires an estimation result of a first mental state of the target person (step S22). The second mental state estimation means 17X estimates a second mental state of the target person, which is a mental state correlated with the first mental state, based on the observation data and the estimation result of the first mental state (step S23).

According to the third example embodiment, the mental state estimation device 1X can accurately estimate the second mental state of the target person based on the estimation result of the first mental state.

### <Other Example Embodiments>

In each of the example embodiments, the manager is, for example, a healthcare (medical care) provider.

In each of the example embodiments, the output control unit 18 may output, to the manager, as information relating to the advice, information indicating a coping method of dealing with the target person based on the estimation result of the mental state (the first mental state or/and the second mental state) of the target person.

According to a first example in this case, the information indicating the correspondence between the estimation result of the mental state and the coping method is stored in the memory 12, the storage device 4 or any device other than the mental state estimation device 1 (not shown). Then, by referring to the information indicating the correspondence, the output control unit 18 determines the coping method to deal with the target person, and outputs information indicating the determined coping method by the output device 3. The term "coping method" herein indicates a remedy for the target person according to the estimated mental state or any other treatment method (including an action to be performed by the target person itself). Examples of "the information indicating the coping method" include any information (name, identification number, content description, or a combination thereof, or the like) for identifying the coping method.

According to a second example, the output control unit 18 may determine the coping method based on a model and the estimation result of the mental state of the target person to thereby output information indicating the determined coping method, wherein the model is generated by machine learning of the correspondence between the estimation result of the mental state and the coping method. In this case, the learned parameters of the above-described model are stored in the memory 12, the storage device 4, or any device other than the mental state estimation device 1 (not shown). When the estimation result of the mental state is obtained, the output control unit 18 inputs the estimation result of the mental state to the model to which the learned parameters are applied, and then determines the coping method based on the information outputted by the model in response to the input, and outputs information indicating the determined coping method by the output device 3.

The method of determining the coping method is not limited to the above-described method. According to the present example embodiment, it is possible to provide information for optimizing an action of the manager who manages the state of the target person, and this leads to improvement of the state of the target person, for example.

In each of the example embodiments, the output control unit 18 may output the observation data (the first observation data or/and the second observation data) affecting the estimation result of the mental state (the estimation result of the first mental state or/and the second mental state) by the output device 3. In this case, for example, if the estimation model which outputs the estimation result of the mental state is based on a neural network, an attention mechanism is added to the above estimation model so that output data prior to the full connection is inputted to the attention mechanism. Then, the output control unit 18 outputs, by the output device 3, the observation data corresponding to the feature values whose coefficient outputted by attention mechanism is equal to or more than a predetermined value as the observation data affecting the estimation result of the mental state. Thus, it becomes possible to provide the manager with information for optimizing the action of the manager. For example, when the manager determines that the outputted estimation result is not appropriate, the manager refers to the observation data that has affected the estimation result of the first mental state or/and the estimation result of the second mental state outputted by the output device 3. Thus, the manager can take actions for improving the measurement state of the biological signal of the sensor 5 related to the observation data on the basis of the referenced observation data.

In the example embodiments described above, the program is stored by any type of a non-transitory computer-readable medium (non-transitory computer readable medium) and can be supplied to a control unit or the like that is a computer. The non-transitory computer-readable medium include any type of a tangible storage medium. Examples of the non-transitory computer readable medium include a magnetic storage medium (e.g., a flexible disk, a magnetic tape, a hard disk drive), a magnetic-optical storage medium (e.g., a magnetic optical disk), CD-ROM (Read Only Memory), CD-R, CD-R/W, a solid-state memory (e.g., a mask ROM, a PROM (Programmable ROM), an EPROM (Erasable PROM), a flash ROM, a RAM (Random Access Memory)). The program may also be provided to the computer by any type of a transitory computer readable medium. Examples of the transitory computer readable medium include an electrical signal, an optical signal, and an electromagnetic wave. The transitory computer readable medium can provide the program to the computer through a wired channel such as wires and optical fibers or a wireless channel.

The whole or a part of the example embodiments (including modifications, the same shall apply hereinafter) described above can be described as, but not limited to, the following Supplementary Notes.

### [Supplementary Note 1]

A mental state estimation device comprising:
an observation data acquisition means configured to acquire observation data obtained by observing a target person;
a first mental state acquisition means configured to acquire an estimation result of a first mental state of the target person; and
a second mental state estimation means configured to estimate a second mental state of the target person, which is a mental state correlated with the first mental state, based on the observation data and the estimation result of the first mental state.

### [Supplementary Note 2]

The mental state estimation device according to Supplementary Note 1,
wherein the first mental state is a mental state which is observed as an appearance of a physiological phenomenon of the target person.

### [Supplementary Note 3]

The mental state estimation device according to Supplementary Note 1,
wherein the first mental state acquisition means is configured to calculate the estimation result, based on first observation data that is at least a part of the observation data and a first estimation model, and
wherein the first estimation model outputs, when the first observation data or a feature value of the first observation data is inputted to the first estimation model, the estimation result of the first mental state at a observing time of the first observation data.

### [Supplementary Note 4]

The mental state estimation device according to Supplementary Note 3,
wherein the first estimation model is a model that is trained based on training data including correct answer data of the first mental state generated based on objective evaluation.

### [Supplementary Note 5]

The mental state estimation device according to Supplementary Note 4,
wherein the estimation result is the correct answer data, and
wherein the second mental state estimation means is configured to estimate the second mental state based on the observation data and the correct answer data.

### [Supplementary Note 6]

The mental state estimation device according to Supplementary Note 1,
wherein the second mental state is a mental state in which a state that is observed as an appearance of a physiological phenomenon of the target person and a state that is not observed as the appearance are mixed.

### [Supplementary Note 7]

The mental state estimation device according to Supplementary Note 1,
wherein the second mental state estimation means is configured to estimate the second mental state based on
   second observation data is at least a part of the observation data,
   the estimation result, and
   a second estimation model, and
wherein the second estimation model outputs, when the estimation result and the second observation data or a feature value of the second observation data are inputted to the second estimation model, the estimation result of the second mental state at an observing time of the second observation data.

### [Supplementary Note 8]

The mental state estimation device according to Supplementary Note 7,
wherein the second estimation model is a model trained based on training data including correct answer data generated based on subjective evaluation.

### [Supplementary Note 9]

The mental state estimation device according to Supplementary Note 1,
wherein the first mental state acquisition means is configured to calculate the estimation result, based on first observation data that is at least a part of the observation data, and
wherein the second mental state acquisition means is configured to calculate the second mental state, based on second observation data that is at least a part of the observation data, and
wherein the second observation data includes the first observation data.

### [Supplementary Note 10]

The mental state estimation device according to Supplementary Note 1,
wherein the second mental state estimation means is configured to estimate a degree of concentration as the second mental state.

### [Supplementary Note 11]

The mental state estimation device according to Supplementary Note 1,
wherein the first mental state and the second mental state are mental states represented by a common index.

### [Supplementary Note 12]

The mental state estimation device according to Supplementary Note 1, further comprising
an output control means configured to output information regarding the second mental state.

### [Supplementary Note 13]

The mental state estimation device according to Supplementary Note 12,
wherein the output control means is configured to output at least one of
   a coping method based on the first mental state and/or
   a coping method based on the second mental state
to optimize actions of at least one of the target person and/or a manager of the target person.

### [Supplementary Note 14]

A mental state estimation method executed by a computer, the mental state estimation method comprising:
acquiring observation data obtained by observing a target person;
acquiring an estimation result of a first mental state of the target person; and
estimating a second mental state of the target person, which is a mental state correlated with the first mental state, based on the observation data and the estimation result of the first mental state.

### [Supplementary Note 15]

A storage medium storing a program executed by a computer, the program causing the computer to
acquire observation data obtained by observing a target person;
acquire an estimation result of a first mental state of the target person; and
estimate a second mental state of the target person, which is a mental state correlated with the first mental state, based on the observation data and the estimation result of the first mental state.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### INDUSTRIAL APPLICABILITY

Examples of the applications include a service related to management (including self-management) based on the mental state.

### DESCRIPTION OF REFERENCE NUMERALS

- 1, 1A, 1X: Mental state estimation device
- 2: Input device
- 3: Output device
- 4: Storage device
- 5: Sensor
- 8: Terminal device
- 100, 100A: Mental state estimation

## Claims

1. A mental state estimation device comprising:
an observation data acquisition means configured to acquire observation data obtained by observing a target person;
a first mental state acquisition means configured to acquire an estimation result of a first mental state of the target person; and
a second mental state estimation means configured to estimate a second mental state of the target person, which is a mental state correlated with the first mental state, based on the observation data and the estimation result of the first mental state.

2. The mental state estimation device according to claim 1,
wherein the first mental state is a mental state which is observed as an appearance of a physiological phenomenon of the target person.

3. The mental state estimation device according to claim 1,
wherein the first mental state acquisition means is configured to calculate the estimation result, based on first observation data that is at least a part of the observation data and a first estimation model, and
wherein the first estimation model outputs, when the first observation data or a feature value of the first observation data is inputted to the first estimation model, the estimation result of the first mental state at an observing time of the first observation data.

4. The mental state estimation device according to claim 3,
wherein the first estimation model is a model that is trained based on training data including correct answer data of the first mental state generated based on objective evaluation.

5. The mental state estimation device according to claim 4,
wherein the estimation result is the correct answer data, and
wherein the second mental state estimation means is configured to estimate the second mental state based on the observation data and the correct answer data.

6. The mental state estimation device according to claim 1,
wherein the second mental state is a mental state in which a state that is observed as an appearance of a physiological phenomenon of the target person and a state that is not observed as the appearance are mixed.

7. The mental state estimation device according to claim 1,
wherein the second mental state estimation means is configured to estimate the second mental state based on
second observation data is at least a part of the observation data,
the estimation result, and
a second estimation model, and
wherein the second estimation model outputs, when the estimation result and the second observation data or a feature value of the second observation data are inputted to the second estimation model, the estimation result of the second mental state at an observing time of the second observation data.

8. The mental state estimation device according to claim 7,
wherein the second estimation model is a model trained based on training data including correct answer data generated based on subjective evaluation.

9. The mental state estimation device according to claim 1,
wherein the first mental state acquisition means is configured to calculate the estimation result, based on first observation data that is at least a part of the observation data, and
wherein the second mental state acquisition means is configured to calculate the second mental state, based on the estimation result and second observation data that is at least a part of the observation data, and
wherein the second observation data includes the first observation data.

10. The mental state estimation device according to claim 1,
wherein the second mental state estimation means is configured to estimate a degree of concentration as the second mental state.

11. The mental state estimation device according to claim 1,
wherein the first mental state and the second mental state are mental states represented by a common index.

12. The mental state estimation device according to claim 1, further comprising
an output control means configured to output information regarding the second mental state.

13. The mental state estimation device according to claim 12,
wherein the output control means is configured to output at least one of
a coping method based on the first mental state and/or
a coping method based on the second mental state
to optimize actions of at least one of the target person and/or a manager of the target person.

14. A mental state estimation method executed by a computer, the mental state estimation method comprising:
acquiring observation data obtained by observing a target person;
acquiring an estimation result of a first mental state of the target person; and
estimating a second mental state of the target person, which is a mental state correlated with the first mental state, based on the observation data and the estimation result of the first mental state.

15. A storage medium storing a program executed by a computer, the program causing the computer to
acquire observation data obtained by observing a target person;
acquire an estimation result of a first mental state of the target person; and
estimate a second mental state of the target person, which is a mental state correlated with the first mental state, based on the observation data and the estimation result of the first mental state.
